# EUROPÄISCHE PATENTANMELDUNG

(11) **EP 2 554 215 A1**
(43) Veröffentlichungstag der Anmeldung: **06.02.2013**
(21) Anmeldenummer: 12175497.2
(22) Anmeldetag: 09.07.2012
(51) Int. Cl.: A61N 1/365, A61B 5/145

(54) **Verfahren und Anordnung zur Reaktivierung eines implantierbaren chemischen Sensors**

(30) Priorität: 03.08.2011 US 201161514490 P
(71) Anmelder: BIOTRONIK SE & Co. KG, 12359 Berlin (DE)
(72) Erfinder: Potschadtke, Jens, 91056 Erlangen (DE)
(74) Vertreter: Lindner-Vogt, Karin L.

(57) **Zusammenfassung**

Verfahren zum Betrieb, insbesondere zur Reaktivierung eines implantierbaren chemischen Sensors, welcher Körperflüssigkeitswerte erfasst, aufweisend:
Benetzen der Sensoroberfläche mit einem Reaktivierungsmittel, das eine chemische Reaktion auf der Sensoroberfläche ausführt, welche auf dem Sensor abgesetzte, aus Körperflüssigkeit stammende Moleküle oder deren Reaktions- oder Abbauprodukte entfernt, und Entnetzen der Sensoroberfläche vom Reaktivierungsmittel, wobei die Sensoroberfläche in situ mit dem Reaktivierungsmittel benetzt und entnetzt wird.

## Beschreibung

Die Erfindung betrifft ein Verfahren zum Betrieb, insbesondere zur Reaktivierung eines implantierbaren Sensors und eine implantierbare Anordnung, die diesen Sensor aufweist.

Die fortlaufende Echtzeitüberwachung von klinischen Analyten, wie z.B. PO₂, PCO₂, K+, Na+, Glucose und Laktat, Gasgehalt, Ionen, Proteinen, Antikörpern, pH-Wert, Hormone, Botenstoffe, Stoffwechselprodukte, Krankheitserreger, Blutbestandteile, Gerinnungsfaktoren, Cholesterin, Nährstoffe, Giftstoffe, Medikamentenbestandteile und weitere Messwerte spielt bei der Gesundheitsvorsorge und bei zahlreichen Therapien eine immer entscheidendere Rolle. Deswegen steigen die Anforderungen an implantierbare chemische Sensoren, die den physiologischen Zustand eines Patienten in Echtzeit bzw. innerhalb des biologischen Umfelds überwachen. Das konkrete Messprinzip eines Sensors hängt stark von der Art der zu messenden Stoffe ab. Ein Sensor kann z.B. ein elektrochemischer Sensor sein, der den pH-Wert misst. Weitere bekannte Sensorprinzipien sind Adsorptionssensoren, die das Adsorbieren von Stoffen (Moleküle oder Ionen) über die Veränderung der Oberflächeneigenschaften erkennen können. Die Oberflächeneigenschaften wiederum können auf vielfältige Weise gemessen werden, zum Beispiel durch mikromechanische Massenbestimmung (messbar beispielsweise über mechanische Resonanz), Kapazitätsänderungen, optische Veränderungen. Solche Sensoren müssen betriebssicher und zuverlässig über längere Zeit unter rauen Bedingungen funktionieren. Trotz vieler Bemühungen bleibt die technische Ausbildung solcher Sensoren im Hinblick auf ihre Widerstandsfähigkeit gegen schädigende Einflüsse im implantierten Zustand nach wie vor eine riesige Herausforderung. Schädigungen dieser Sensoren ergeben sich hauptsächlich durch die unerwünschten Wechselwirkungen zwischen der Sensoroberfläche und dem biologischen Medium.

Beispiele dieser Wechselwirkungen sind die Ablagerung von Proteinen oder die Bindung bzw. Aktivierung von metabolischen aktiven Zellen (Blutplättchen) auf der Sensoroberfläche. Die Adsorption von Bluttplättchen ändert zum Beispiel die chemische Umgebung des Implantats durch Aufnahme von O₂ und Abgabe von CO₂, die zu einer Reduzierung des gemessenen pH-Wertes führt.
Die in subkutanen Geweben implantierten Sensoren führen hingegen zu Zellenbindung und Sehnengewebeverkapselung, als Antwort des Selbstverteidigungssystems des Körpers.
Als Folge davon muss Sensorik, die im dauerhaften Kontakt zu Körperflüssigkeiten steht, regelmäßig aufwändig gereinigt und ggf. sogar ganz oder teilweise ersetzt werden, was neben dem hohen Aufwand mit zeitweiser Nichtverfügbarkeit der Sensorinformationen verbunden ist.
Das Messprinzip eines chemischen Sensors, welches etwa in der Adsorption von bestimmten Molekülen oder deren Reaktions- oder Abbauprodukte besteht, kann auch generell einer Langzeitverwendung entgegenstehen. Ohne weitere Maßnahmen wäre ein solcher Sensor nur für eine einmalige Messung zu gebrauchen. Um den Sensor wieder verwendbar zu machen müssen die adsorbierten Moleküle wieder entfernt werden.

Eine bekannte Lösung sieht die Benutzung von mehreren anfangs gekapselten Sensoren vor. Hierbei wird je nach Bedarf ein neuer kalibrierter Sensor in Betrieb genommen, wenn ein geschädigter Sensor zu deaktivieren ist. Die Verkapselung der neu in Betrieb genommenen Sensoren wird nach Stilllegung der verbrauchten Sensoren jeweils gezielt entfernt. Es versteht sich, dass auch diese Lösung hohen technischen Aufwand erfordert und möglicherweise unökonomisch ist, weil der Sensor mehrfach vorgehalten werden muss und üblicherweise bei Implantaten Beschränkungen bezüglich der zur Verfügung stehenden Volumina und Oberflächen greifen. Da man Implantate nicht beliebig groß machen kann, ergibt sich eine starke Beschränkung der Anzahl der vorzuhaltenden Sensoren, welches wiederum die Dauernutzung oder die Anzahl der Messungen beschränkt.

Es ist daher die Aufgabe der Erfindung, ein Verfahren und eine entsprechende implantierbare Anordnung anzugeben, die die oben genannten Probleme löst.

Diese Aufgabe wird durch ein Verfahren mit den Merkmalen des Anspruchs 1 und durch eine implantierbare Anordnung mit den Merkmalen des Anspruchs 8 gelöst. Zweckmäßige Fortbildungen des Erfindungsgedankens sind Gegenstand der jeweiligen abhängigen Ansprüche.

Die Erfindung schließt den grundlegenden Gedanken ein, ein Verfahren zur Reaktivierung und optional auch Kalibrierung eines implantierbaren chemischen Sensors, welcher Körperflüssigkeitswerte erfasst, im implantierten Zustand und im Wesentlichen bei laufendem Betrieb anzugeben. Sie schließt weiter den Gedanken ein, die erforderliche Reinigung der relevanten Sensoroberfläche (also derjenigen Oberfläche, mit der der Sensor die informationsrelevante Wechselwirkung mit der umgebenden Körperflüssigkeit hat) durch Benetzen der Sensoroberfläche mit einem Reaktivierungsmittel, das eine chemische Reaktion auf der Sensoroberfläche ausführt, welche auf dem Sensor abgesetzte, aus Körperflüssigkeit stammende Moleküle oder deren Reaktions- oder Abbauprodukte entfernt oder umwandelt, und Entnetzen der Sensoroberfläche vom Reaktivierungsmittel zu bewirken. Das erfindungsgemäße Verfahren zeichnet sich also dadurch aus, dass die Sensoroberfläche in situ mit einem Reaktivierungsmittel benetzt und von diesem entnetzt wird.

Alternativ oder zusätzlich zum Reaktivierungsmittel kann der Sensor auch mit einer Kalibrierflüssigkeit in Kontakt gebracht werden. Mittels der bekannten chemischen und physikalischen Eigenschaften der Kalibrierflüssigkeit kann der Sensor kalibriert werden. Die Kalibrierflüssigkeit ist jeweils auf das Messprinzip des Sensors abgestimmt. Bei einem pH-Sensor kann beispielsweise eine gepufferte Lösung mit bekanntem pH-Wert als Kalibrierflüssigkeit dienen. Bei geeigneter geometrischer Anordnung können auch mehrere Kalibrierflüssigkeiten gezielt in Kontakt mit dem Sensor gebracht werden und so sogar eine Mehrpunktkalibration zu ermöglichen.

Diese Erfindung ermöglicht eine reversible Reaktivierung und optional auch Kalibrierung des implantierten chemischen Sensors, ohne dass dieser aus dem Körper des Patienten entnommen wird. Durch diese Reaktivierung ist der Sensor dauernutzbar, was eine wichtige Vorraussetzung für die Nutzung in einem Implantat ist.

In einer Ausführungsform kann das Benetzen der Sensoroberfläche durch Elektrobenetzung (Electrowetting) erfolgen. Diese Technik erlaubt es, die Oberflächenspannung von leitfähigen Flüssigkeiten mittels eines elektrischen Feldes zu verändern. Somit kann die Sensoroberfläche unter elektrischer Ansteuerung mit einem Reaktivierungsmittel und/oder einer Kalibrierflüssigkeit gezielt benetzt werden.

Des Weiteren kann durch eine geeignete geometrische Anordnung von Elektroden und einer geeigneten zeitlichen Beschaltung von Elektroden die Elektrobenetzung dazu genutzt werden, Tropfen oder allgemeiner die Grenzfläche zwischen einem flüssigen Elektrolyt und einer mit ihm nicht vermischbaren Flüssigkeit oder einem Gas zu bewegen. Somit kann zum Beispiel ein Sensor in Kontakt mit einem Elektrolyten gebracht werden oder der Elektrolyt von ihm entfernt werden.

Alternativ kann das Reaktivierungsmittel und/oder einer Kalibrierflüssigkeit durch andere Methoden, z.B. durch die Wirkung mindestens eines mechanischen Aktors, in Kontakt mit der Sensoroberfläche gebracht und wieder entfernt (bzw. die Kontaktfläche des Reaktivierungsmittels mit der Sensoroberfläche wesentlich vergrößert und anschließend wieder verkleinert) werden. Somit wird die relativ aufwendige Erzeugung von für die Elektrobenetzung konfigurierten elektrischen Feldern entbehrlich.

Als ein mechanischer Aktor im Sinne der Patentanmeldung kann ein Piezo-Aktor, ein elektromagnetischer Aktor, ein Bimetall-Aktor, ein Hydraulik- oder Pneumatikaktor, ein Formgedächtnis-Aktor oder ein magnetostriktiver Aktor eingesetzt wird.

In einer zweckmäßigen Ausführungsform des Verfahrens wird mindestens eine teildurchlässige Außenmembran zwischen der Sensoroberfläche und der Körperflüssigkeit angebracht, und dazwischen befindet sich eine mit der Sensoroberfläche in direktem Kontakt stehende Koppelflüssigkeit. Somit ist die Koppelflüssigkeit über die Außenmembran an die Körperflüssigkeit gekoppelt, um den Sensor gegen direkte Einwirkung der Körperflüssigkeit zu schützen. Als Vorteil sind geringere Anforderungen an die Biokompatibilität des Sensors bzw. des Reaktivierungsmittels gegeben, weil kein direkter Kontakt Sensor-Körperflüssigkeit besteht. Da hierbei das Reaktivierungsmittel und/oder die Kalibrierflüssigkeit im gleich bleibenden Volumen zwischen der Membran und der Sensoroberfläche liegt, funktioniert das Benetzen und Entnetzen der Sensoroberfläche genauer und zuverlässiger.
Gemäß einer weiteren Ausgestaltung dieser Ausführungsform kann das Volumen zwischen der Außenmembran und der Sensoroberfläche mit mehreren Flüssigkeiten gefüllt sein. Bei diesen Flüssigkeiten handelt es sich um mindestens eine Koppelflüssigkeit, mindestens ein Reaktivierungsmittel und wahlweise eine oder mehrere Trennflüssigkeiten. Die Flüssigkeiten stehen miteinander in Kontakt, das heißt sie berühren sich gegenseitig, jedoch vermischen sich diese wegen unterschiedlicher physikalischer und/oder chemischer Eigenschaften nicht. Dadurch wird das Bewegen dieser Flüssigkeiten durch Elektrobenetzung ermöglicht. Einer der genannten mechanischen Aktoren kann dabei eine Druckänderung im Volumen zwischen der Außenmembran und der Sensoroberfläche bewirken, um die Molekül - oder Ionenkonzentration von Flüssigkeiten im Innenraum über Osmose und Umkehrosmose zu verändern.

In einer weiteren Ausführungsform wird noch eine teildurchlässige Innenmembran zwischen der Außenmembran und der Sensoroberfläche in der Koppelflüssigkeit angebracht. Durch die Wirkung eines mechanischen Aktors kann hierbei eine Druckänderung an der Innenmembran bewirkt werden. Somit kann die Molekül- oder Ionenkonzentration innerhalb des zwischen der Außenmembran und Innenmembran und zwischen der Innenmembran und der Sensoroberfläche eingeschlossenen Volumens über Osmose und Umkehrosmose gezielt verändert werden.

In einer weiteren Ausführungsform wird noch eine weitere teildurchlässige Membran mit wahlweise anderen Eigenschaften zwischen Innenraum und Außenraum angebracht. Der Innenraum wird vollständig mit Elektrolyten (eine oder mehrere zusammenhängende Flüssigkeitsmengen) und einer oder mehreren weiteren nicht mischbaren Flüssigkeitsmengen (z.B. Silikonöl) aufgefüllt. Durch geschickte Anwendung von Elektrobenetzung kann dann selektiv Öl oder Elektrolyt in Kontakt mit den Membranen gebracht werden. Durch die Wirkung eines mechanischen Aktors (z.B. Piezo) kann eine Druckänderung im Innenraum erzeugt werden. Die Druckänderung kann dann im Zusammenhang mit der selektiven Benetzung der Membranen mit Elektrolyt oder Öl dazu verwendet werden, um mittels Osmose und Umkehrosmose gezielt die Molekül- oder Ionenkonzentration in den Elektrolytflüssigkeiten zu verändern.

In einer weiteren Ausführung ist ein Reaktivierungsmittel und/oder eine Kalibrierflüssigkeit an die Außen- und/oder Innenmembranoberflächen gekoppelt. Durch Elektrobenetzung oder die Wirkung eines mechanischen Aktors kann die Außen- und/oder der Innenmembranoberfläche benetzt und entnetzt werden, um auf den Membranoberflächen abgesetzte, aus der Körperflüssigkeit stammende Moleküle oder deren Reaktions- oder Abbauprodukte zu entfernen. Somit kann sowohl der Sensor als auch die schützende Membran dauernutzbar sein.

Falls mindestens eine erste und eine zweite nebeneinander liegende Außen- und/oder Innenmembran zwischen der Sensoroberfläche und der Körperflüssigkeit vorhanden sind, kann die Wirkung mindestens einer Membran durch das Verhindern der Molekül- oder Ionenübertragung durch die Membran beim Benetzen, z.B. über Elektrobenetzung, der entsprechenden Membranoberfläche desaktiviert werden. Vorteilhaft können die Membranen unterschiedliche Eigenschaften aufweisen, so dass unterschiedliche Moleküle oder Ionen durch die Membrane gelangen können. Das kann nützlich sein, wenn zum Beispiel der Sensor Körperflüssigkeitswerte in unterschiedlichen biologischen Umfeldern überwachen muss.

Zusätzlich oder alternativ zur Regeneration kann ein Benetzen und Entnetzen der Sensoroberfläche mit einer speziellen Flüssigkeit mit bekannten Eigenschaften erfolgen, um eine Kalibration des Sensors durchzuführen.
Die Erfindung schließt ferner den Gedanken ein, eine implantierbare Anordnung mit einem Sensor, einem an die Sensoroberfläche gekoppelten Reaktivierungsmittel, das eine geeignete Reinigungs-Reaktion auf der Sensoroberfläche ausführt, und einem Mittel zum Benetzen und Entnetzen der Sensoroberfläche mit dem Reaktivierungsmittel zu bilden. Die Erfindung ermöglicht unter diesem Aspekt die Bereitstellung einer implantierbaren Anordnung, in der ein chemischer Sensor über längere Zeitintervalle immer betriebsfähig ist. Das Reaktivierungsmittel erlaubt eine Reaktivierung der Sensoroberfläche durch z.B. ein Abspülen bzw. eine Verdrängung und/oder Umwandlung von chemischen Liganden auf der Sensoroberfläche. Bei definierten Eigenschaften des Reaktivierungsmittels kann ferner der Sensor während der Benetzung kalibriert werden.

In einer Ausführungsform der Erfindung weisen die Mittel zum Benetzen und Entnetzen eine erste Elektrode, welche an das Reaktivierungsmittel gekoppelt ist und eine zweite Elektrode auf, die an den Sensor gekoppelt ist, wobei beide zusammen die Benetzung der Sensoroberfläche durch Elektrobenetzung ermöglichen. Grundsätzlich umfassen die Mittel zum Benetzen und Entnetzen also ein Elektrodenpaar, bei dem der Platz zwischen den Elektroden mit einer biokompatiblen Flüssigkeit, die dem Reaktivierungsmittel entspricht, gefüllt ist. Diese Flüssigkeit kann Silikonöl oder eine andere hydrophobe Flüssigkeit sein.

Eine Variante besteht darin, dass das Reaktivierungsmittel eine erste und eine zweite Flüssigkeit mit verschiedenen chemischen und/oder physikalischen Eigenschaften aufweist, wobei die erste Flüssigkeit durch Elektrobenetzung bewegt wird und die zweite Flüssigkeit (Reagens) die chemische Reaktion auf der Sensoroberfläche ausführt. Hierbei kann die erste (benetzende) Flüssigkeit eine Hülle (z.B. um einen Tropfen der zweiten Flüssigkeit) bilden. Wird die Hülle durch Elektrobenetzung bewegt, so bewegt sich auch die darin enthaltene Flüssigkeit. Somit kann man spezielle Flüssigkeiten verwenden, die zum Zweck der Elektrobenetzung bzw. der chemischen Reaktion mit der Sensoroberfläche jeweils besser geeignet sind.

Alternativ zur Elektrobenetzung kann man die Sensoroberfläche mit dem Reaktivierungsmittel durch andere Methoden, z.B. eines mechanischen Aktors, beispielsweise unter Ausnutzung des piezoelektrischen Effekt, benetzen und entnetzen, wobei die praktische Vorteile des elektrischen Betriebs ebenfalls zur Geltung kommen.

In einer Ausführung der implantierbaren Anordnung kann das Reaktivierungsmittel, welches die Sensoroberfläche benetzt, ein freier Tropfen sein oder eine Flüssigkeit sein, die in einem Behälter, insbesondere einem Spalt, einer Rinne oder einer Kapillare, enthalten ist, gegebenenfalls an ein Reservoir des Reaktivierungsmittels gekoppelt sein. Durch Elektrobenetzung oder beispielsweise piezoelektrischem Effekt kann das Reaktivierungsmittel die Sensoroberfläche gezielt benetzen. Um die Messung der Körperflüssigkeitswerte zu ermöglichen, ist der Behälter mit mindestens einer Öffnung im Bereich der Sensoroberfläche versehen. Alternativ kann die Öffnung mit einer oder mehreren teildurchlässigen Membranen geschützt werden, so dass die Sensoroberfläche nicht in direktem Kontakt mit der Körperflüssigkeit steht. Selbstverständlich stellen sich hierbei die gleichen Wirkungen und Vorteile der Membranen heraus, wie oben erklärt.

Bezüglich der vorgenannten Verfahrensaspekte ergeben sich auch einige weitere Vorrichtungsaspekte. Beispielsweise weist der Sensor mindestens eine teildurchlässige Außenmembran zwischen der Sensoroberfläche und der Körperflüssigkeit, und eine mit der Sensoroberfläche in direktem Kontakt stehenden Koppelflüssigkeit auf, die über die Außenmembran an die Körperflüssigkeit gekoppelt ist, um den Sensor gegen direkte Einwirkung der Körperflüssigkeit zu schützen, wobei das Reaktivierungsmittel im gleich bleibenden Volumen zwischen der Membran und der Sensoroberfläche liegt.

Dabei kann die eine oder mehrere teildurchlässige Außenmembran als eine geometrische Anordnung mehrerer verschiedener Außenmembransegmente gebildet sein, die zusätzlich differierende chemische und / oder physikalische Eigenschaften aufweisen. Durch Bewegen der Flüssigkeiten im Volumen zwischen der Außenmembransegmenten und der Sensoroberfläche werden sie Segmente mit einer Flüssigkeitssorte in Kontakt gebracht, für die sie durchlässig beziehungsweise nicht durchlässig sind, und somit die Wirkung der Membransegmente selektiv aktiviert und deaktiviert werden.

Ebenso ist es denkbar, den Sensor mit mindestens einer teildurchlässigen Innenmembran zwischen der mindestens einen Außenmembran und der Sensoroberfläche in der Koppelflüssigkeit und mit einem an die Innenmembran gekoppelten mechanischen Aktor zu versehen, um die Molekül- oder Ionenkonzentration innerhalb des zwischen der Außenmembran und Innenmembran und zwischen der Innenmembran und der Sensoroberfläche geschlossenen Volumens über Osmose und Umkehrosmose zu verändern.

Zusätzlich kann ein an die Membranoberflächen gekoppeltes zusätzliches Reaktivierungsmittel zum Benetzen und Entnetzen der Außen- und/oder der Innenmembranoberfläche durch Elektrobenetzung oder die Wirkung eines mechanischen Aktors vorgesehen sein, um auf den Membranoberflächen abgesetzte aus der Körperflüssigkeit stammende Moleküle oder deren Reaktions- oder Abbauprodukte zu entfernen.

Ebenso kann zusätzlich ein Reservoir für die verbrauchte Reaktivierungsflüssigkeit vorgesehen sein.

Weitere Vorrichtungsaspekte der Erfindung ergeben sich ohne weiteres aus den weiteren oben erläuterten Verfahrensaspekten, so dass hier auf eine erneute Erläuterung dieser Aspekte verzichtet werden kann.

Der in dem obigen Verfahren und der implantierbaren Anordnung eingesetzte Sensor kann ein beliebiger chemischer Sensor sein, der Körperflüssigkeitswerte erfasst, wie zum Beispiel ein pH-Sensor, Proteinsensor, elektrochemischer Sensor, Ionenkonzentrations-Sensor, enzymatischer Sensor, mikromechanischer Sensor, optischer Sensor oder interferometrischer Sensor handeln. Denkbar sind auch Sensoren, die nach anderen Wirkprinzipien funktionieren, wie beispielsweise zur Messung von Blutzucker, Sauerstoffsättigung, CO2 Gehalt, Gasgehalt, Ionen, Proteinen, Antikörpern, pH-Wert, Hormone, Botenstoffe, Stoffwechselprodukte, Krankheitserreger, Blutbestandteile, Gerinnungsfaktoren, Cholesterin, Nährstoffe, Giftstoffe oder Medikamentenbestandteile.

Vorteile und Zweckmäßigkeiten der Erfindung ergeben sich im Übrigen aus der nachfolgenden Beschreibung ausgewählter Ausführungsbeispiele anhand der Figuren. Von diesen zeigen:
- Fig. 1: eine schematische Darstellung einer implantierbaren Anordnung gemäß der Erfindung,
- Fig. 2a und 2b: schematische Darstellungen einer implantierbaren Anordnung mit einem Elektrodenpaar als Mittel zum Benetzen und Entnetzen gemäß einer Ausführungsform der Erfindung,
- Fig. 3a und 3b: schematische Darstellungen einer implantierbaren Anordnung mit einem Piezo-Aktor als Mittel zum Benetzen und Entnetzen gemäß einer alternativen Ausführungsform der Erfindung,
- Fig. 4: eine schematische Darstellung einer implantierbaren Anordnung mit einer ersten und einer zweiten Flüssigkeit als Reaktivierungsmittel gemäß einer weiteren Ausführungsform der Erfindung,
- Fig. 5: eine schematische Darstellung einer implantierbaren Anordnung mit einem Behälter und Reservoir für das Reaktivierungsmittel gemäß einer noch weiteren Ausführungsform der Erfindung, und
- Fig. 6a und 6b: schematische Darstellungen einer implantierbaren Anordnung mit einer oder mehreren teildurchlässigen Membranen zwischen der Sensoroberfläche und der Körperflüssigkeit gemäß einer anderen Ausführungsform der Erfindung.
- Fig. 7a und b: schematische Darstellung einer implantierbaren Anordnung mit geschlossenen innerem Volumen, einer Membran und Koppelflüssigkeit, einem Behälter und Reservoir für das Reaktivierungsmittel gemäß einer noch weiteren Ausführungsform der Erfindung.
- Fig. 8: schematische Darstellung einer implantierbaren Anordnung mit geschlossenen innerem Volumen, einer Membran und Koppelflüssigkeit, einem Behälter und Reservoir für das Reaktivierungsmittel, einem Behälter und Reservoir für verbrauchte Reaktivierungsmittel gemäß einer anderen Ausführungsform der Erfindung.

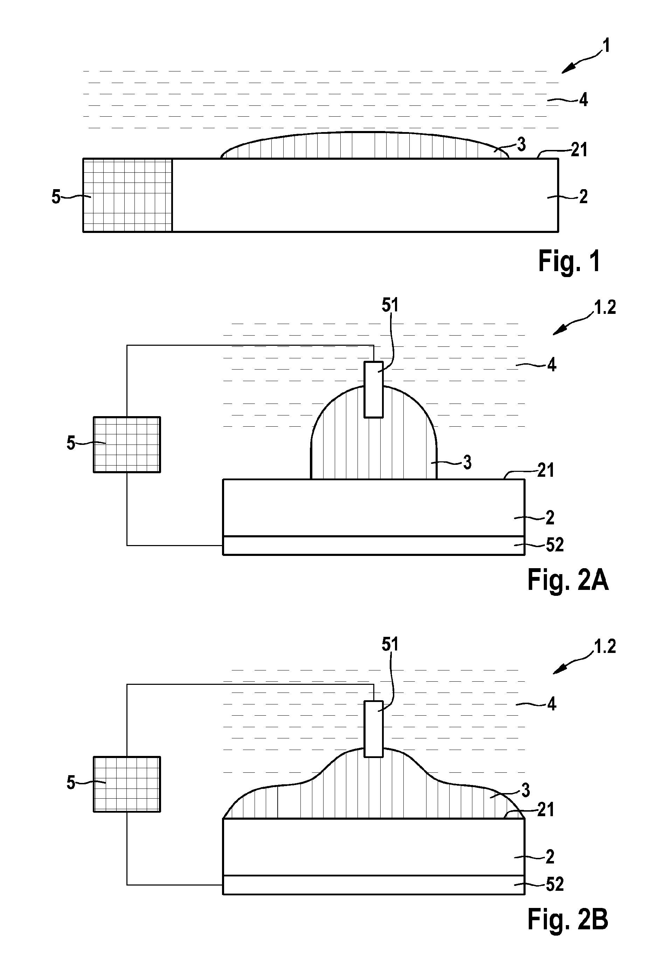

Fig. 1 zeigt eine implantierbare Anordnung 1, mit einem Sensor 2, der eine Sensoroberfläche 21 aufweist, welche mit der Körperflüssigkeit 4 eines Patienten in Kontakt steht. An der Sensoroberfläche 21 ist ein Reaktivierungsmittel 3 vorgesehen, das eine chemische Reaktion auf der Sensoroberfläche 21 ausführt. Die implantierbare Anordnung 1 weist ferner ein Mittel 5 zum Benetzen und Entnetzen der Sensoroberfläche 21 mit dem Reaktivierungsmittel 3 auf, um auf dem Sensor abgesetzte, aus Körperflüssigkeit stammende Moleküle zu entfernen.

Fig. 2a und 2b zeigen eine implantierbare Anordnung 1.2, in der das Benetzen und Entnetzen der Sensoroberfläche 21 mit dem Reaktivierungsmittel 3 durch Elektrobenetzung erfolgt. Das Mittel 5 zum Benetzen und Entnetzen weist eine an das Reaktivierungsmittel 3 gekoppelte erste Elektrode 51 und eine an den Sensor gekoppelte zweite Elektrode 52 auf. Das Reaktivierungsmittel 3 ist hierbei ein freier Tropfen. Mittels Wirkung eines elektrischen Feldes wird die Oberflächespannung des Reaktivierungsmittels 3 geändert, so dass dieses von einem ersten Zustand, in dem es nur einen Teil der Sensoroberfläche 21 bedeckt (Fig. 2a), in einen zweiten Zustand, in dem es die ganze Sensoroberfläche 21 gezielt benetzt (Fig. 2b), überführt wird.

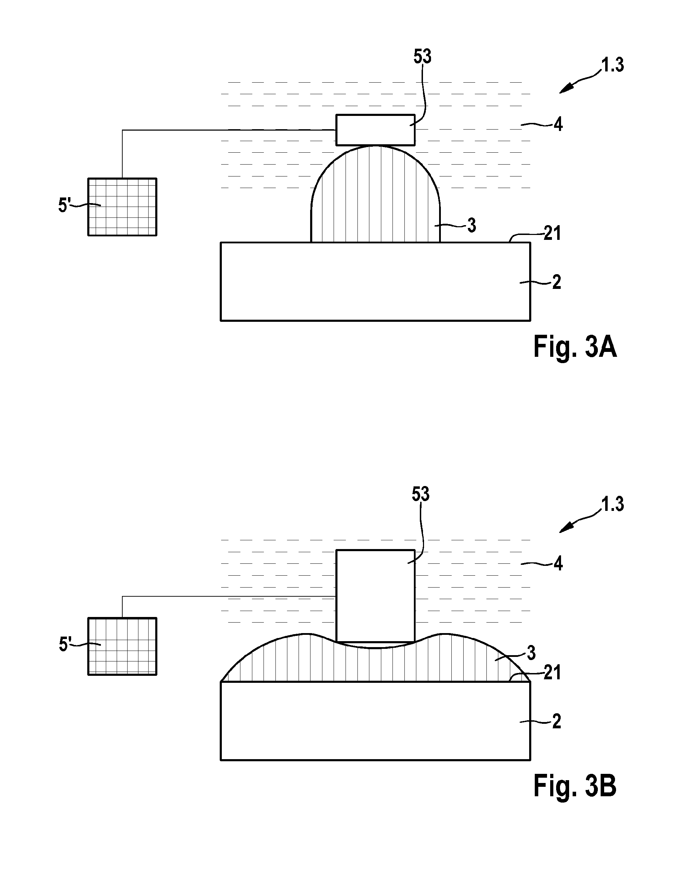

Fig. 3a und 3b zeigen als Alternative der implantierbaren Anordnung 1.2 der Figuren 2a und 2b eine weitere Anordnung 1.3, in der das Benetzen und Entnetzen der Sensoroberfläche 21 mit dem Reaktivierungsmittel 3 durch die Wirkung des piezoelektrischen Effektes erfolgt. Ein Mittel 5' zum Benetzen und Entnetzen weist einen an das Reaktivierungsmittel 3 gekoppelten Piezo-Aktor 53 auf. Das Reaktivierungsmittel 3 ist auch hierbei ein freier Tropfen. Auf das Anlegen einer elektrischen Spannung reagiert der Piezo-Aktor 53 mit einer mechanischen Verformung. Hierdurch wird das Reaktivierungsmittel-Tropfen 3 derart deformiert, dass die Sensoroberfläche 21 von diesem gezielt benetzt wird (Fig. 3b).

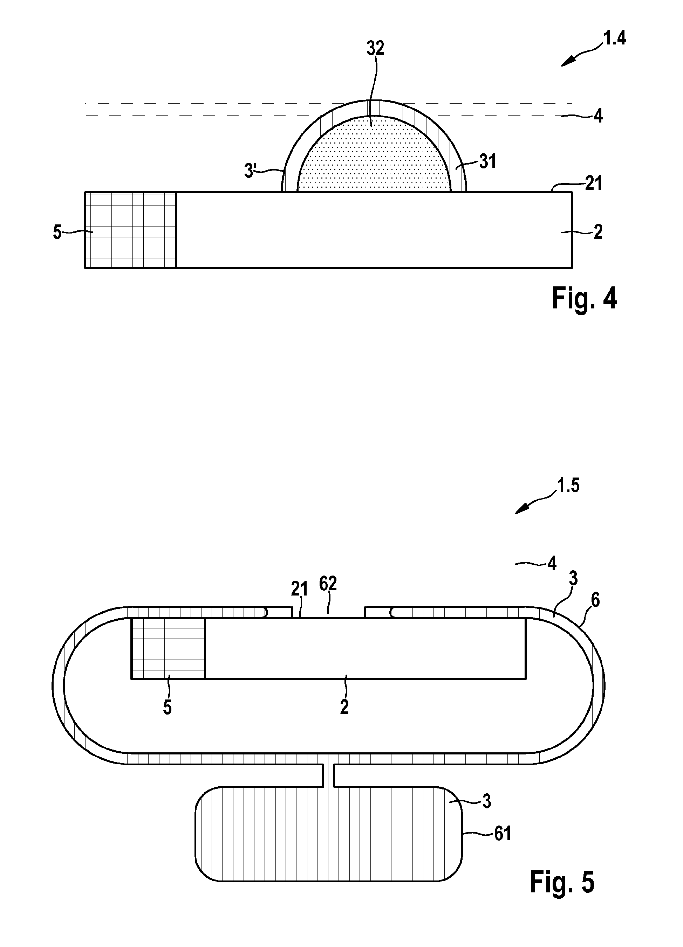

Fig. 4 zeigt eine weitere implantierbare Anordnung 1.4, wobei ein Reaktivierungsmittel 3' eine erste und eine zweite Flüssigkeit 31, 32 aufweist. Insbesondere bildet die erste Flüssigkeit 31 eine Hülle um einen Tropfen der zweiten Flüssigkeit 32. Während die erste Flüssigkeit 31 nur für die Elektrobenetzung Verwendung findet, dient die zweite Flüssigkeit lediglich der chemischen Reaktion auf der Sensoroberfläche 21. Wird die Hülle durch Elektrobenetzung bewegt, so bewegt sich auch die darin enthaltene Flüssigkeit, die dann die chemische Reaktion auf der Sensoroberfläche 21 gezielt ausführt.

Fig. 5 zeigt eine weitere implantierbare Anordnung 1.5, wobei das Reaktivierungsmittel 3 in einer Kapillare 6 enthalten ist. An diese Kapillare 6 ist ein Reservoir 61 gekoppelt, dass das Reaktivierungsmittel 3 enthält. Im Bereich der Sensoroberfläche 21 ist die Kapillare 6 mit einer Öffnung 62 versehen, um die Körperflüssigkeit 4 mit der Sensoroberfläche 21 in Verbindung zu bringen.

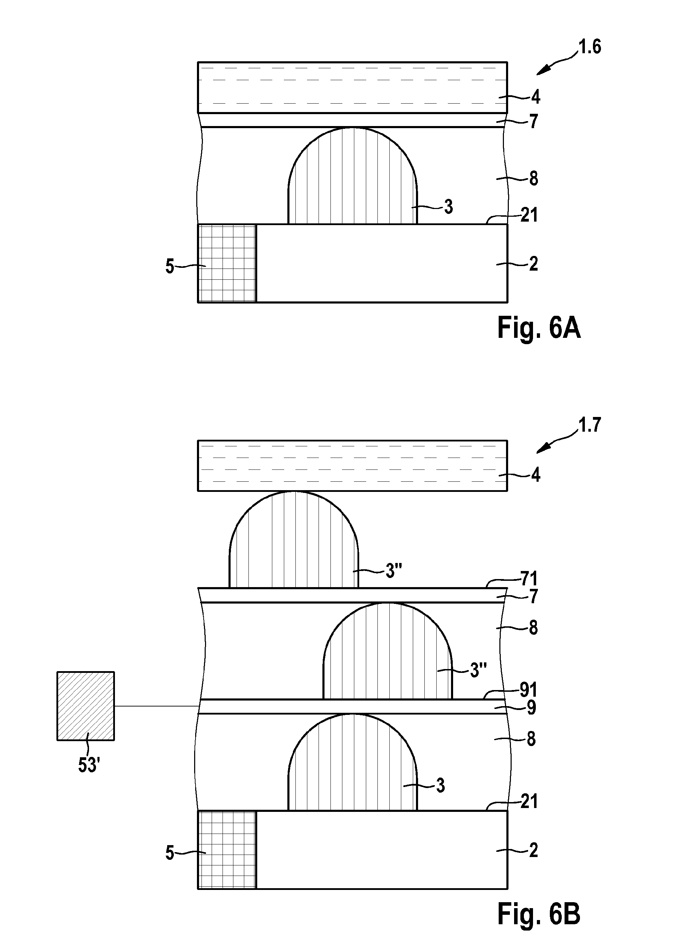

Fig. 6a zeigt eine weitere implantierbare Anordnung 1.6, in der eine teildurchlässige Membran 7 (Außenmembran) zwischen der Sensoroberfläche 21 und der Körperflüssigkeit 4 angeordnet ist. Hierbei ist eine Koppelflüssigkeit 8 vorhanden, die mit der Sensoroberfläche 21 in direktem Kontakt steht und über die Membran 7 an die Körperflüssigkeit 4 gekoppelt ist. Daraus folgt, dass die Sensoroberfläche 21 nicht in direktem Kontakt mit der Körperflüssigkeit 4 steht und das Reaktivierungsmittel 3 im gleich bleibenden Volumen zwischen der Membran 7 und der Sensoroberfläche 21 liegt.

Fig. 6b zeigt als Anordnung 1.7 eine Variante der implantierbaren Anordnung gemäß Figur 6a, in der eine zusätzliche teildurchlässige Membran 9 (Innenmembran) zwischen der Außenmembran 7 und der Sensoroberfläche 21 in der Koppelflüssigkeit 8 angeordnet ist, wobei ein Piezo-Aktor 53' an die Innenmembran 9 gekoppelt ist, der eine Druckänderung an der Innenmembran 9 erzeugt. Ein zusätzliches Reaktivierungsmittel 3" ist hierbei an die Membranen 7, 9 zum Benetzen und Entnetzen der Außen- und Innenmembranoberflächen 71, 91 gekoppelt.

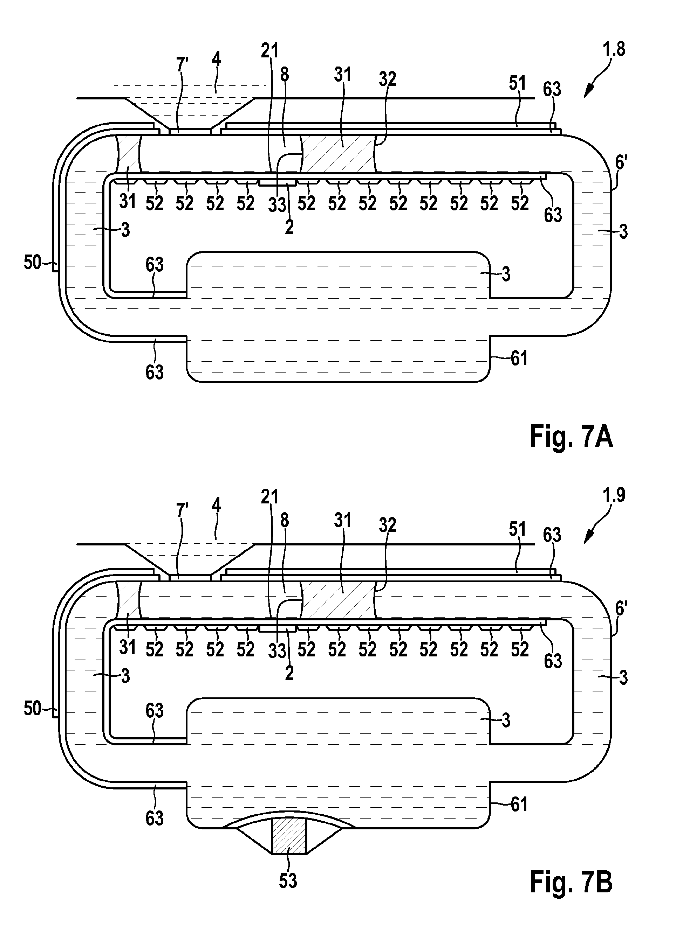

Fig 7a zeigt eine Anordnung 1.8, bei der ein geschlossener Flüssigkeitskreislauf 6 mit einem Reservoir 61 gekoppelt ist und wiederum der Flüssigkeitskreislauf an einer Stelle über eine teildurchlässige Membran 7 an die Körperflüssigkeit gekoppelt ist. Der Flüssigkeitskreislauf 6 ist nun so gefüllt, dass sich vier Bereiche ergeben, die abwechselnd mit einem Elektrolyten (3 und 8) und einer mit ihm nicht vermischbaren nicht elektrisch leitenden Trennflüssigkeit 31 (z.B. Silikonöl) gefüllt sind.

Entlang des Flüssigkeitskreislaufs sind nun in geeigneter geometrischer Anordnung und gegebenenfalls mehrfacher Ausführung Elektroden 50-52 angebracht. Die Oberfläche der Elektroden ist mit einer dünnen Schicht 63 hydrophob gemacht worden. Durch gezielte Beschaltung der Elektrode 50-52 mit Spannung kann die Benetzung der Oberfläche 63 direkt oberhalb der Elektroden an der Grenzfläche 32 zwischen Elektrolyt und Nicht-Elektrolyt so verändert werden, dass die Grenzfläche 32 sich gezielt in eine Richtung bewegt. Mit ihr bewegt sich die dann die gesamte Flüssigkeitssäule 3, 31, 8, 31 innerhalb des geschlossenen Kreislaufs 6.

Je nach Position der Grenzfläche 32 können folgende Konfigurationen A-D erreicht werden: A) Sensor 2 ist mit Koppelflüssigkeit 8 über die teildurchlässige Membran 7 and die Körperflüssigkeit 4 gekoppelt. Die Trennflüssigkeit 31 trennt die Koppelflüssigkeit von der Flüssigkeit 3. B) Sensor 2 ist vollständig von Flüssigkeit 31 umgeben, wodurch gegebenenfalls eine Reinigung oder Kalibration des Sensors durchgeführt werden kann. Je nach geometrischer Anordnung der teildurchlässigen Membran 7 kann diese entweder mit Flüssigkeit 8 oder der Trennflüssigkeit 31 in Kontakt sein. C) Sensor 2 ist vollständig von Flüssigkeit 3 umgeben, welche wiederum eine Reinigungs- oder Regenrationswirkung haben kann. Membran 7 ist von Trennflüssigkeit 31 umgeben, so dass ein Austausch von Stoffen der Flüssigkeit 3 mit der Körperflüssigkeit 4 verhindert werden kann. D) Sowohl Sensor 2 als auch Membran 7 sind in Kontakt mit Flüssigkeit 3, wodurch gegebenenfalls eine Reinigungs- oder Regenerationswirkung erzielt wird.
Fig 7b zeigt die Anordnung 1.9 als eine Abwandlung von Anordnung 1.8, bei der innerhalb des geschlossenen inneren Volumens ein Druck erzeugender mechanischer Aktor 53 angebracht ist, der dazu verwendet wird, in Kombination mit der teildurchlässigen Membran 7 Osmose und Umkehrosmose zu bewirken.

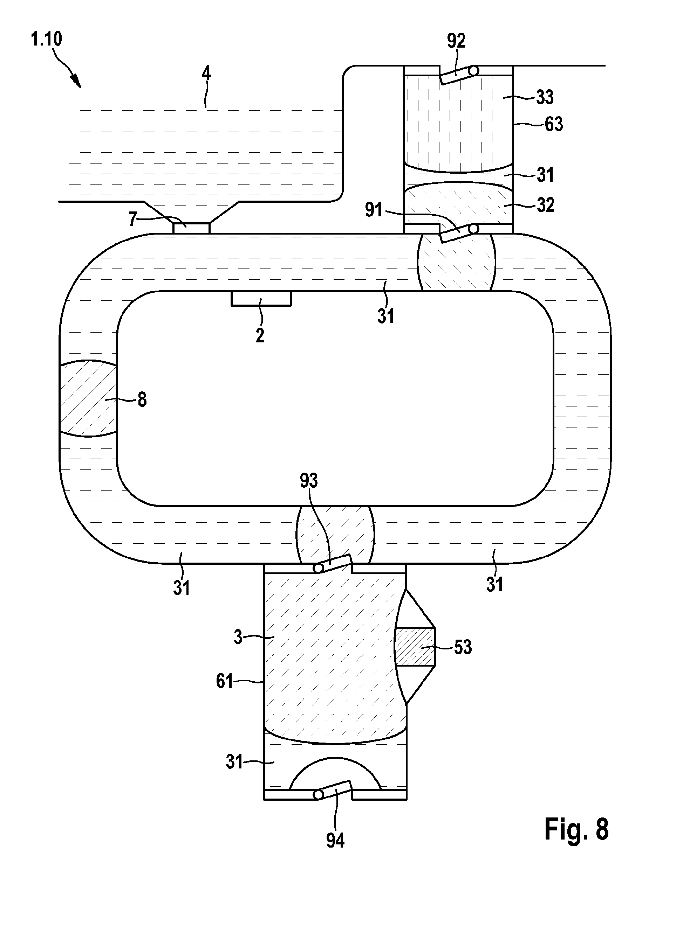

Fig 8 zeigt die Anordnung 1.10 als eine Abwandlung von Anordnung 1.9, bei der innerhalb des geschlossenen inneren Volumens ein durch ein Ventil 91 abgeschlossenes Reservoir 63 für verbrauchte Reaktivierungsflüssigkeit 32 befindet. Als Druckausgleichselement wird eine biokompatible nicht mischbare Flüssigkeit 31 (zum Beispiel Silikonöl) oder ein weiteres Ventil 92 genommen. Zur Aufnahme von verbrauchter Reaktivierungsflüssigkeit kann eine biokompatible Flüssigkeit (z.B. Kochsalzlösung) über Ventil 92 in die Umgebung abgelassen werden. In der gezeigten Ausführung besitzt auch das Reservoir 61 ein Ventil 94 und eine Trennflüssigkeit 31, um den Druckausgleich mit dem Außenbereich durchführen zu können. Ventil 93 regelt den Austritt der Regenerierflüssigkeit 3 in den Innenraum.

Durch eine geeignete Anordnung von Elektroden (in diesem Schaubild der Übersichtlichkeit halber nicht gezeigt) kann die gesamte Flüssigkeitssäule bestehend aus den Flüssigkeiten 3, 31, 32 und 8 über Elektrobenetzung bewegt werden und die verschiedenen Flüssigkeiten gezielt in die Position an den Ventilen oder dem Sensor 2 gebracht werden um dann über den mechanischen Aktor 53 gezielt Flüssigkeiten durch die jeweiligen Ventile zu bewegen.

Die Ausführung der Erfindung ist nicht auf die oben beschriebenen Beispiele und hervorgehobene Aspekte beschränkt, sondern ebenso in einer Vielzahl von Abwandlungen möglich, die im Rahmen fachgemäßen Handelns liegt.

## Patentansprüche

1. Verfahren zum Betrieb, insbesondere zur Reaktivierung eines implantierbaren chemischen Sensors, welcher Körperflüssigkeitswerte erfasst, aufweisend:
Benetzen der Sensoroberfläche mit einem Reaktivierungsmittel, das eine chemische oder physikalische Reaktion auf der Sensoroberfläche ausführt, welche auf dem Sensor abgesetzte, aus Körperflüssigkeit stammende Moleküle oder deren Reaktions- oder Abbauprodukte entfernt oder umwandelt, und
Entnetzen der Sensoroberfläche vom Reaktivierungsmittel,
wobei die Sensoroberfläche in situ mit dem Reaktivierungsmittel benetzt und entnetzt wird.

2. Verfahren nach Anspruch 1, wobei das Benetzen der Sensoroberfläche durch Elektrobenetzung oder durch die Wirkung mindestens eines mechanischen Aktors erfolgt.

3. Verfahren nach einem der Ansprüche 1 oder 2, ferner aufweisend:
Anbringen mindestens einer teildurchlässigen Außenmembran zwischen der Sensoroberfläche und der Körperflüssigkeit, und einer mit der Sensoroberfläche in direktem Kontakt stehenden Koppelflüssigkeit, die über die Außenmembran an die Körperflüssigkeit gekoppelt ist, um den Sensor gegen direkte Einwirkung der Körperflüssigkeit zu schützen, wobei das Benetzen und Entnetzen der Sensoroberfläche und/oder der Außenmembran mit dem im gleich bleibenden Volumen zwischen der Außenmembran und der Sensoroberfläche liegenden Reaktivierungsmittel erfolgt.

4. Verfahren nach Anspruch 3, ferner aufweisend:
eine Füllung des Volumens zwischen der Außenmembran und der Sensoroberfläche mit mehreren Flüssigkeiten, davon mindestens eine Koppelflüssigkeit, mindestens ein Reaktivierungsmittel und wahlweise eine oder mehrere Trennflüssigkeiten, die sich gegenseitig berühren, aber wegen unterschiedlicher physikalischer und/oder chemischer Eigenschaften nicht vermischen, wobei das Bewegen dieser Flüssigkeiten durch Elektrobenetzung bewerkstelligt wird.

5. Verfahren nach Anspruch 3 oder 4, ferner aufweisend:
das Bewirken einer Druckänderung im Volumen zwischen der Außenmembran und
der Sensoroberfläche durch einen mechanischen Aktor, um die Molekül- oder Ionenkonzentration von Flüssigkeiten im Innenraum über Osmose und Umkehrosmose zu verändern.

6. Verfahren nach einem der Ansprüche 3 bis 5, ferner aufweisend:
Anbringen mindestens einer teildurchlässigen Innenmembran zwischen der Außenmembran und der Sensoroberfläche in der Koppelflüssigkeit, und
Bewirken einer Druckänderung an der Innenmembran durch einen mechanischen Aktor, um die Molekül- oder Ionenkonzentration innerhalb des zwischen der Außenmembran und Innenmembran und zwischen der Innenmembran und der Sensoroberfläche geschlossenen Volumens über Osmose und Umkehrosmose zu verändern.

7. Verfahren nach einem der Ansprüche 3 bis 6, ferner aufweisend:
Benetzen und Entnetzen der Außen- und/oder der Innenmembranoberfläche mit einem an die Membranoberflächen gekoppelten zusätzlichen Reaktivierungsmittel durch Elektrobenetzung oder die Wirkung eines mechanischen Aktors, um auf den Membranoberflächen abgesetzte, aus der Körperflüssigkeit stammende Moleküle oder deren Reaktions- oder Abbauprodukte zu entfernen.

8. Implantierbare Anordnung mit einem Sensor, welcher Körperflüssigkeitswerte erfasst, einem an der Sensoroberfläche gekoppelten Reaktivierungsmittel, das eine chemische Reaktion auf der Sensoroberfläche ausführt, durch die auf dem Sensor abgesetzte, aus Körperflüssigkeit stammende Moleküle oder deren Reaktions- oder Abbauprodukte entfernt werden, und einem Mittel zum Benetzen und Entnetzen der Sensoroberfläche mit dem Reaktivierungsmittel, wobei das Mittel zum Benetzen und Entnetzen vorzugsweise einen mit dem Reaktivierungsmittel gekoppelten mechanischen Aktor aufweist..

9. Anordnung nach Anspruch 8, wobei das Mittel zum Benetzen und Entnetzen eine an das Reaktivierungsmittel gekoppelte erste Elektrode und eine an den Sensor gekoppelte zweite Elektrode zur Elektrobenetzung aufweist, wobei das Reaktivierungsmittel vorzugsweise eine biokompatible Flüssigkeit, insbesondere Silikonöl, aufweist.

10. Anordnung nach Anspruch 9, wobei das Reaktivierungsmittel eine erste und eine zweite Flüssigkeit mit verschiedenen chemischen und/oder physikalischen Eigenschaften aufweist, wobei die erste Flüssigkeit durch Elektrobenetzung bewegt wird und die zweite Flüssigkeit die chemische Reaktion auf der Sensoroberfläche ausführt und wobei die erste Flüssigkeit die zweite Flüssigkeit hüllt, so dass die zweite Flüssigkeit durch die Bewegung der ersten Flüssigkeit mitbewegt wird.

11. Anordnung nach einem der Ansprüche 8 bis 10, wobei das Reaktivierungsmittel als freier Tropfen vorliegt

12. Anordnung nach einem der Ansprüche 8 bis 11, ferner mit einem Behälter, insbesondere einem Spalt, einer Rinne oder einer Kapillare, welcher das Reaktivierungsmittel enthält und an ein Reservoir des Reaktivierungsmittels gekoppelt ist.

13. Anordnung nach einem der Ansprüche 8 bis 12, ferner mit mindestens einer teildurchlässigen Außenmembran zwischen der Sensoroberfläche und der Körperflüssigkeit und einer mit der Sensoroberfläche in direktem Kontakt stehenden Koppelflüssigkeit, die über die Außenmembran an die Körperflüssigkeit gekoppelt ist, um den Sensor gegen direkte Einwirkung der Körperflüssigkeit zu schützen, wobei das Reaktivierungsmittel im gleich bleibenden Volumen zwischen der Membran und der Sensoroberfläche liegt.

14. Anordnung nach Anspruch 13, mit mindestens einer teildurchlässigen Innenmembran zwischen der Außenmembran und der Sensoroberfläche in der Koppelflüssigkeit, und einem an die Innenmembran gekoppelten mechanischen Aktor, um die Molekül- oder Ionenkonzentration innerhalb des zwischen der Außenmembran und Innenmembran und zwischen der Innenmembran und der Sensoroberfläche geschlossenen Volumens über Osmose und Umkehrosmose zu verändern.

15. Anordnung nach Anspruch 14, mit einem an die Membranoberflächen gekoppelten zusätzlichen Reaktivierungsmittel zum Benetzen und Entnetzen der Außen - und/oder der Innenmembranoberfläche durch Elektrobenetzung oder die Wirkung eines mechanischen Aktors, um auf den Membranoberflächen abgesetzte aus der Körperflüssigkeit stammende Moleküle oder deren Reaktions- oder Abbauprodukte zu entfernen.
